# EUROPEAN PATENT SPECIFICATION

(11) **EP 0 497 870 B1**
(45) Date of publication and mention of the grant of the patent: **16.09.1998**
(21) Application number: 90916544.1
(22) Date of filing: 25.10.1990
(51) Int. Cl.: G01N 33/569, C12Q 1/00, B01L 3/00

(54) **ENZYME ASSAY AND ASSAY KIT TO MEASURE CELLULAR ACTIVATION**
ENZYMTEST UND TESTSATZ ZUR MESSUNG ZELLULÄRER AKTIVIERUNG
ANALYSE ENZYMATIQUE ET ENSEMBLE D'ANALYSE POUR MESURER L'ACTIVATION CELLULAIRE

(30) Priority: 25.10.1989 US 426294
(43) Date of publication of application: 12.08.1992
(73) Proprietor: JAFFE, Russell M., Reston, VA 22091 (US)
(72) Inventor: JAFFE, Russell M., Reston, VA 22091 (US)
(74) Representative: Mallalieu, Catherine Louise
(86) International application number: US9006214
(87) International publication number: WO9106859

(56) References cited:
- US-A- 4 528 267
- US-A- 4 592 997
- ROSE N. et al., "Methods in Immuno-diagnosis", published 1980 by John Wiley & Sons (N.Y.), see pages 41-43
- FISHER SCIENTIFIC, Catalog published 1988, see page 1306
- B. DAVIS et al., "Microbiology" published 1973. see pages 56-57, 643-652
- JOURNAL OF IMMUNOLOGICAL METHODS, vol. 61, issued 1983, M. MATTES et al., "Preparing Monolayers of Non-Adherent Mammalian cells", pages 145-150, see entire document.
- JOURNAL OF IMMUNOLOGICAL METHODS, vol. 65, issued 1983, T. MOSMANN, "Rapid Colorimeric Assay foir cellular Growth and Survival: Application to Proliferation and Cytotoxicity Assays", pages 55-63, see entire document
- "Culture of Animal Cells", by R.I. Freshney, published by A. Liss, New York, NY 10011, p.59-62

## Description

### BACKGROUND OF THE INVENTION

### Field of the Invention

The invention in the field of immunology, cell biology, and medicine, relates to enzymatic assays and assay kits for measuring the activation of cells, especially blood cells such as lymphocytes, by cell-activating substances such as antigens to which the lymphocytes are specifically reactive.

### Description of the Background Art

The measurement of specific immune responses or general reactivity of the immune system is a valuable tool for diagnosis and study of a large number of important diseases. Originally, such tests were limited to in vivo techniques such as skin tests to detect the presence of immediate or delayed hypersensitivity.

Since the 1960's, numerous in vitro assays which serve as correlates of in vivo immune responses have been developed. Many of these bioassays are based on the fact that cells of the immune system, namely lymphocytes, recognize chemical structures to which they respond, such as an antigen for which they are specific. As part of this response, lymphocytes are activated, and enlarge into "blasts," divide, and/or to differentiate.

Thus, in vitro measurements of lymphocyte blastogenesis or lymphocyte proliferation in the presence of appropriately presented challenge antigens have served as measures of specific immunological responsiveness, mainly of the T lymphocyte class. Similar reactions induced in a larger proportion of lymphocytes by polyclonal activators, which stimulate entire classes of lymphocytes (rather than just antigen-specific populations), have enabled physicians and investigators to assess the general reactivity of lymphocyte populations.

Polyclonal activation is induced by a variety of substances, such as mitogenic lectins derived from plants (e.g. concanavalin A, phytohemagglutinin, pokeweed mitogen), bacterial products (e.g. lipopolysaccharides, cell wall substances, enterotoxins), and various chemicals or biochemicals (e.g. phorbol esters, sodium periodate, galactose oxidase, dextran sulfate). (See, Roitt, I. et al., IMMUNOLOGY, C.V. Mosby Co. (1985)).

As currently practiced, in vitro assays of lymphocyte activation or stimulation entail the measurement of cell growth (i.e. proliferation) by incorporation of radioactive precursors of DNA, most typically ³H-thymidine or ¹²⁵Iododeoxyuridine, into proliferating cells. These assays are technically demanding, requiring sterile cell culture conditions and multistep fractionation of blood samples, making them relatively expensive. In addition, these assays generate radioactive waste which is becoming an increasing problem in the United States. Perhaps most importantly, these assays usually require 3-7 days, and results can be quite variable, especially if the difference in response (for example, between a patient and a healthy control) is modest, though significant, rather than "all -or-none." Furthermore, to measure responses to a battery of stimuli, current assays require relatively large numbers of lymphocytes, which presents a problem when assaying lymphopenic or leukopenic patients (for example after chemotherapy, bone marrow transplantation, or in immunodeficiency diseases such as AIDS)

Therefore, an assay for lymphocyte activation which is rapid, simple, requires little blood per sample, and avoids the use of radioisotopes would be of great utility. For clinical laboratory purposes, it would be important that such assays: (1) be easily routinized, requiring as little cell handling as possible; (2) be automated, and (3) provide more reproducible results than current cell proliferation assays allow.

One approach to a more rapid assay would be to measure an event which occurs at an earlier time after the initial stimulus to cell activation, that precedes DNA synthesis and cell proliferation. The "activation" of intracellular or membrane-associated enzymes, is one type of event which occurs early after the cell membrane is "triggered" by a signal, such as an antigen contacting a specific lymphocyte (Sell, S., Immunology, Immunopathology, and Immunity, Elsevier, 1987). The activation, or availability for response, of such enzymes can be easily monitored employing enzyme-substrate reactions with chromogenic substrates or other physically detectable reactants or products.

Landegren, U. (J. Immunol. Meth. 67:379-388 (1984)) took advantage of a ubiquitous lysosomal enzyme, hexosaminidase, and developed an assay to quantitate the number of cells present in a microplate well. A chromogenic substrate for this enzyme, p-nitro-phenol-N-acetyl-β-D-glucosaminide, was used to generate a colored reaction product which was measured as the absorbance at 405 nm (A₄₀₅) using a colorimetric plate reader. When the substrate was added to cells in culture, the amount of color generated (i.e. reaction product formed) was directly proportional to the cell number for a given reaction time. This method was applied to measure lymphocyte proliferation in response to growth factors, the adherence of cells to a surface, and adherence of cells to plates coated with antibodies. Thus, while possibly providing a useful substitute for the radioisotopes used in assays of lymphocyte proliferation, the method as taught in this reference would not be of value in measuring early lymphocyte activation, since this enzyme was apparently active and available to the substrate at all times. A further problem was the presence of this enzyme in serum, requiring thorough washing of blood cells to remove serum-associated enzyme prior to their incubation in vitro.

The assay described by Landegren (supra) was modified by Alcina, A., et al. (J. Immunol. Meth. 105:1-8 (1987) to detect viability and killing of intracellular parasites.

Mosmann, T. (J. Immunol. Meth. 65:55-63 (1983)) developed a similar assay using the tetrazolium salt MTT (3-(4,5-dimethylthiazol-2-yl)-2,5-diphenyl tetrazolium bromide), which is modified by mitochondrial dehydrogenase enzymes to form a blue formazan product, the absorbance of which can be measured spectrophotometrically at 570 nm. Over a certain range of cell numbers, this substrate resulted in a linear relationship between cell number and color formed. The method has been useful in measuring the stimulation of growth of several lymphocyte cell lines by growth factors. Others have modified the method of Mosmann to measure the survival of cells in cytotoxicity assays (Green L.M., et al., J. Immunol. Meth. 70:257-268 (1984)).

Alkaline phosphatase is an enzyme known to be associated with secretory and absorptive surfaces of various normal and leukemic lymphocyte types (Neumann, H. et al., Proc. Natl. Acad. Sci. USA 73:1432 (1976); Culvenor, J.G., et al., J. Immunol. 126:1974 (1981)). Garcia-Rozas, C., et al. (J. Immunol. 129:52-55 (1982)) assayed alkaline phosphatase in mouse lymphocytes cultured in vitro with various mitogens, using p-nitrophenylphosphate as substrate. Prior to assay the cells were fixed with glutaraldehyde and treated with detergent, after which the substrate was added, and the color reaction read directly in the plates (A₄₀₅) using a plate reader. It was found that alkaline phosphatase was associated particularly with B lymphocytes. Enzyme activity was significantly elevated in B cells "activated" for 3 days with lipopolysaccharide (LPS) or pokeweed mitogen (PWM), and was associated with enlarged "blast" cells.

Hashimoto, N., et al., (J. Immunol. Meth. 90:97-103 (1986)) modified the assay of Garcia-Rozas et al. (supra), by adding the substrate in detergent directly to microwells containing stimulated B cells (after one wash of the cells). The assay allowed measurement of B cell proliferation after 3 days of stimulation with LPS and 1-2 days of further stimulation with T cell-derived factors. The assay was advantageous in that, in contrast to measurement of ³H-thymidine uptake, it could selectively detect B cell proliferation even in the presence of high numbers of "contaminating" T cells, and could measure cells in HAT medium (commonly used to select hybridomas). No evidence was presented indicating any enzyme "activation" prior to 3 days, and the threshold of detectable enzyme activity of fresh B cells appeared to require 10⁵ or more cells.

EPO Publication 0166505 (2 January 1985) provides a process of quantitative detection of alkaline phosphatase via chromatographic separation and colorimetric assay, wherein 2 x 10⁻⁶ units of enzyme activity gave a detectable signal. However, the assay system and kits disclosed in this publication are designed for use with partially purified enzymes, not whole cells.

Chan, K-M., et al. (Anal. Biochem. 157:375-380 (1986)), described a direct colorimetric assay for measuring Ca²⁺-stimulated ATPase activity on adipocyte plasma membrane preparations or liver microsomes. The assay depended upon the coupling of a chromogenic reaction to the Pᵢ product generated from the ATP substrate. The enzyme was measured only in cell extracts, and use with whole cells was not suggested.

EPO publication 0122028 (17 October 1984) discloses a colorimetric assay useful for detecting an enzyme in a biological specimen. A substrate is absorbed to a surface such as a swab, which is contacted with the enzyme-containing specimen to produce a color reaction on the surface. This assay system is purely qualitative rather than quantitative, and was particularly designed for the detection of the presence of bacteria in a sample. The assay system was designed to detect the presence of one or more bacteria types in a sample by dipping a swab, to which one or more substrates has been absorbed, into the bacteria-containing sample and detecting the color reaction in the swab.

### SUMMARY OF THE INVENTION

The present invention is based on the observation that upon activation of cells, such as blood cells, with cell-activating substances, such as a specific antigen, certain cellular enzymes are activated or otherwise become available for reaction with a substrate. The inventor has provided a rapid, simple, reproducible assay to measure this reaction using intact cells, in which a substrate is converted into a detectable product or a product which is coupled to another reaction yielding a detectable product.

According to one aspect the invention relates to an in vitro method of detecting the response of intact animal cells to an extra-cellular, exogenous cell activator substance, being a substance which, when in contact with the intact cells, activates an intracellular enzyme, which method comprises contacting the cells in vitro in the presence of a substrate for the activated enzyme with the said activator substance immobilised onto a solid support and detecting the consequent activity of the activated enzyme in the presence of the enzyme substrate, such contact taking place in one or more wells of a microtiter plate, characterised in that the well or wells of the microtiter plate in which the contact takes place between the intact cells, the immobilised activator and the enzyme substrate has or have (each of them) a vertical side wall height of from 1 to 2 mm and the method is carried out such that a) the ratio of the internal diameter of the well (in mm.) to the volume of the sample (in µl.) is at least 0.1:1, and b) the ratio of the vertical height of the well to the vertical height (depth) of the sample in the well is in the range 1.1:1 to 5:1.

The method of the present invention may be used to detect the presence of lymphocytes specific for a given antigen or antigens, by: (a) bringing the lymphocytes into contact with an antigen or antigens for a suitable interval, wherein interaction with the antigen causes an enzyme of the lymphocytes to become available for reaction; (b) providing an enzyme substrate capable of reacting with the enzyme; and (c) detecting the activation of the lymphocytes by measuring a product of the enzyme-substrate reaction.

The method of the present invention may also be used to detect immunological sensitization to an antigen in a subject by: (a) providing blood cells from the subject to the antigen; (b) incubating said cells with the antigen for a period sufficient to allow the cells to undergo activation, which causes an enzyme of the cells to become available for reaction; (c) providing an enzyme substrate capable of reacting with the enzyme; and (d) detecting the immunological sensitization by measuring a product of the enzyme-substrate reaction.

According to another aspect, the invention provides a microtiter plate for use in the method of the present invention wherein the well or wells of the microtiter plate contain therein intact animal cells and an immobilised exogenous cell activator substance which, when in contact with the intact cells, activates an intracellular enzyme characterised in that the well or wells of the microtiter plate in which the contact takes place between the intact cells, the immobilised activator and the enzyme substrate has or have (each of them) a vertical side wall height of from 1 to 2 mm and the method is carried out such that a) the ratio of the internal diameter of the well (in mm.) to the volume of the sample (in µl.) is at least 0.1:1, and b) the ratio of the vertical height of the well to the vertical height (depth) of the sample in the well is in the range 1.1:1 to 5:1.

According to a further aspect, the invention provides an assay kit for use in the present method comprising the microtiter plate of the present invention wherein the well or wells of the microtiter plate in which the contact takes place between the intact cells, the immobilised activator and the enzyme substrate has or have (each of them) a vertical side wall height of from 1 to 2 mm and the method is carried out such that a) the ratio of the internal diameter of the well (in mm.) to the volume of the sample (in µl.) is at least 0.1:1, and b) the ratio of the vertical height of the well to the vertical height (depth) of the sample in the well is in the range 1.1:1 to 5.1.

The kit may be used for measuring the activation of cells, such as blood cells. in which the kit provides a cell-activating substance such as an antigen, bound to a support matrix, and a substrate for the enzyme.

### DESCRIPTION OF THE PREFERRED EMBODIMENTS

The assay of the invention involves incubating cells under test with a cell-activating substance which has been bound in advance to a support matrix with which the cells are then brought into contact. The cell-activating substance is preferably bound to the bottom and sidewalls of the well. The cells and activating substance are allowed to interact for a sufficient period of time to allow activation of an enzyme or enzymes in the cell. A major advantage of this invention is the rapidity with which the activation can be measured (optimally, about 3 hours). A substrate for the activated enzyme is added with the cells or after the activation has progressed, and is acted upon by the active or available enzyme to result in a detectable reaction product which signals the activation of the cells.

By the term "cell-activating substance" is intended a substance which by virtue of a cell's possession of receptors or other binding structures or uptake mechanisms, and/or the appropriate metabolic machinery, can trigger a reaction in the cell.

For cells of the immune system such as lymphocytes and monocytes/macrophages, cell-activating substances include specific antigens. The antigen may be a peptide, a glycopeptide, a lipo-protein, or a hapten. Included among these antigens are the particular class known as allergens, many of which are haptens. Cell activating substances for cells of the immune system also include immune complexes, complement components, immunoglobulin molecules fragments, lymphokine and other cytokines (e.g. IL1, IL2, IL4 etc.). Allergens serve as activating agents for specific lymphocytes, or for basophils or mast cells bearing specific IgE antibodies on their surface. Food substances, which may also be allergens, can also serve as cell-activating substances as intended here, as can a variety of environmental chemicals. For a discussion of activation of cells of the immune system, see, for example, Roitt, I. et al., IMMUNOLOGY, C.V. Mosby Co. (1985).

A list of substances which can activate blood cells as detected by the assay of this invention are listed in Table 1, below. This list is not intended to be exhaustive, and one of skill in the art will be able to select additional activating agents for use in this invention.

The antigens or other cell activating agent of this invention can be allowed to bind to the support matrix, either by simple pre-incubation or by chemical coupling as is well known in the art.

Fat cells can be activated by essential fatty acids, arachidonic acid and its metabolites, or triglycerides.

Erythroid cell precursors and other blood cells can be activated by chemicals found in the environment including mercury salts and sulfites such as metabisulfites, etc.

Liver cells can be activated by arachidonic acid, essential fatty acids, and a variety of xenobiotics.

Glucose-6-phosphate polymers can be used to activate a variety of cells, and detect a deficiency or hyperactivity of glucose-6-phosphate dehydrogenase.

A variety of cells can be activated by hormones or growth factors. Various cell types and various biological molecules or xenobiotics that are capable of activating them are well-known in the art (see Smith, E.L. et al., PRINCIPLES OF BIOCHEMISTRY: Mammalian Biochemistry, 7th Ed., McGraw-Hill, (1983).

**TABLE 1**

| SUBSTANCES FOR DETERMINATION OF IMMUNOLOGICAL HYPERSENSITIVITY BY ASSAY OF CELL ACTIVATION | |
|---|---|
| CATEGORY | ANTIGEN (OR EXTRACT) |
| A. FOODS | |
| 1.Crustacean/ Mollusk | crab, lobster, shrimp, clam, oyster, scallop |
| | |
| 2. Dairy | butter, cheese, milk, casein, yogurt |
| | |
| 3. Fish | anchovy, bass, catfish, codfish, haddock, perch/mackerel, red snapper ,salmon, sardine, sole/flounder/halibut, swordfish, trout, tuna, turbot/whitefish |
| | |
| 4. Fowl | egg white, egg yolk, chicken, goose, duck, turkey |
| | |
| 5. Fruit | apple, apricot, banana, berries (blackberry, blueberry, boysenberry, cranberry, raspberry, strawberry), cherry, coconut, currant, date, fig, grape/raisin, grapefruit, kiwi, lemon, lime, mandarin/tangerine, mango, melon (cantaloupe, honeydew, watermelon), nectarine, orange, papaya, peach, pear, pineapple, plum, tamarind |
| | |
| 6. Grain | amaranth, barley, buckwheat, corn, millet, oats, rice (white), rice (brown), rye, triticale, wheat |
| | |
| 7. Meat | beef/veal, lamb/mutton, pork, deer/venison, rabbit |
| | |
| 8. Nut/Seed | alfalfa, almond, anise, brazil nut, cashew, chestnut, hazelnut, macadamia, peanut, pecan, pine, pistachio, poppy seed, pumpkin, sesame, sunflower, walnut |
| | |
| 9. Oils | cod liver, corn, cottonseed, hazelnut, hydrogenated oil, linseed, olive, peanut, primrose, safflower, sesame, sunflower, walnut |
| | |
| 10. Spice | allspice, arrowroot, bay leaf, caraway seed, chili pepper, cinnamon, clove, curry, dill, ginger, horse-radish, mace, mustard, m\nutmeg, oregano, paprika, parsley, pepper (black, cayenne, white, bell), peppermint, rosemary, sage/basil, spearmint, thyme, vanilla |
| 11. Vegetable | artichoke, asparagus, avocado, beans (black-eyed peas, carob, chick pea, kidney, lima, navy, pinto, soya string/wax), beet, broccoli, brussels sprout, cabbage, carrot, cauliflower, celery, corn, cucumber, eggplant, garlic, lentil, lettuce, mushroom, olive, onion, parsley, parsnip, red pepper, green pepper, pimento, potato (white, sweet), radish, rhubarb, rutabaga, spinach, squash, turnip, watercress |
| | |
| MISCELLANEOUS | algae (spirulina), coffee, cocoa, cola, gin (juniper berries), hops, kelp/seaweed, malt, psyllium seed, rose hips, tapioca, tea, tobacco, tofu/miso, yeast (Baker's, Brewer's) |
| | |
| FOOD ADDITIVES & PRESERVATIVES | Aspartame, BHT/BHA, food coloring, monosodium glutamate, saccharine, benzoate, sulfite/metabisulfite |
| | |
| CHEMICALS & DRUGS | acetaminophen, aldehyde (formaldehyde), baking powder, baking soda (sodium bicarbonate), caffeine, carbamates, coal tar, detergents, halogenated pesticides, metallic catalysts (Ni, Cd, Hg, etc.), nitrates, organophosphates, phenol, petroleum byproducts (solvents), salicylate, soaps (sodium dodecyl sulfate), |

By the term "enzyme activation" is intended any of a number of changes which results in measurable increase in enzyme activity when prior to such activation there was little or no detectable enzyme activity. Thus, a cell-activating substance, by reacting with a cell, can trigger a number of intracellular events which result in the alteration of an enzyme from a chemically inactive to a chemically active form, such as by phosphorylation or dephosphorylation, or by allosteric changes in the active site. Alternatively, enzymes can be translocated from a site inaccessible to the substrate to a site accessible to the substrate, such as from the cytoplasm to the cell surface. Intracellular changes in the localization of an enzyme, such as from inside a membrane vesicular structure to the outside, can make an enzyme available to a substrate which is in the cytoplasm. Enzyme activation as used in this invention also includes the enzyme being made available to the substrate by virtue of a change in the cell allowing the substrate to enter and come in contact with the enzyme as part of the process of cell activation. Such changes in enzyme activity, localization, and the cell's permeability or uptake of substrate materials are well known in the art, and are described in detail in Alberts, B. et al., MOLECULAR BIOLOGY OF THE CELL (2nd Edition), Garland Publishing, Inc., 1989.

By the term "substrate" is intended: (1) a substrate which is acted upon directly by the enzyme of interest to produce a detectable product (e.g. a chromophore); (2) a combination of a first substrate and a second substance, wherein the first substrate yields a product which when coupled with a second enzyme/substrate reaction, or with a second coupled dye (such as a diazo dye), yields a product which is detectable and reflects the activity of the enzyme of interest. The second substance may be a co-enzyme precursor plus a chromogenic precursor, wherein the enzyme of interest reacts with the co-enzyme precursor, such as, for example nicotinamide adenine dinucleotide phosphate (NADP), under suitable conditions to produce NAD, which is then employed in a series of cyclic chemical reactions forming a generation catalyst which acts to produce a detectable product from a precursor (such as a chromogenic precursor). The generation catalyst can be another enzyme, e.g. diaphorase, as a reduction catalyst. The absorbance of a chromophore so generated is measured by conventional colorimetric techniques.

A cyclic NAD/NADH reaction with different oxidation-reduction catalysts for phosphatase determination is disclosed in EPO Publication 0058539 (published 25 August 1982).

Chromophore precursors useful in this invention include tetrazolium salts such as 2-(p-iodophenyl),3-(p-nitrophenyl)-5-phenyl-tetrazolium chloride (INT); 3-(4,5-dimethylthiazol-2-yl)-2,5-diphenyl tetrazolium bromide (MTT); 2,2'5,5'-tetra-(p-nitrophenyl )-3,3'-(3,3'-dimethoxy-4,4'-diphenylene) diphenyltetrazolium chloride (TNBT); 2,2'-di(p-nitrophenyl)-5,5'-diphenyl-3,3'-(3,3'-dimethoxy-4,4'-diphenylene) diphenyl tetrazolium chloride (NBT); 2,2-diphenylene-3,3'5,5'-tetraphenyl ditetrazolium chloride (neotetrazolium chloride or NT); 2,3-5-triphenyltetrazolium chloride (TT); and the like.

Such chromophore precursors can act at various sites distal to the enzymatic reaction of interest. For example, lactic dehydrogenase (LDH) activity, in the presence of lactate, causes reduction of NAD to NADH, which can reduce a tetrazolium salt such as those described above, to a reduced formazan, which can be detected colorimetrically at the appropriate wavelength. The amount of formazan formed is a measure of LDH activity.

Alternatively, alkaline phosphatase activity results in generation of NAD from NADP. The NAD can couple to the LDH reaction described above and result in similar formazan formation, which in this case would be a measure of alkaline phosphatase activity.

Clearly, in intact cells, such enzymatic reactions proceed concomitantly, and with the appropriately selected cell-activating substances and chromophore precursors, can interact to increase the sensitivity of the assay.

Phorbol esters are particularly well suited as both generalized cell activating substances and as substrates for esterases such as phospholipase. By conjugating chromophore precursors to phorbol esters such as phorbol myristate acetate (PMA), the single compound provides the activating signal, and is subsequently cleaved by the activated enzyme to yield a chromogenic product which can be detected. In the assays of hypersensitivity to food and environmental chemical antigens, the phorbol ester conjugate, phorbol-12-retinoate-13-acetate serves as the "positive control."

The enzymes which are activated under conditions of cell-activation and are assayed in this invention include: arylhydrolases such as arylsulfatases, kinases, lipases, phosphatases (e.g. alkaline phosphatase), esterases glycosidases, hexosaminidase, peptidases, and nucleases (e.g. DNase, RNase), esterases, oxidases (such as mixed oxidases) or dehydrogenases (e.g. LDH).

Substrates for these enzymes which either contain detectable labels such as chromophores, or are coupled to additional reactants, are listed in Table 2, below, which is not intended to be a limiting or exhaustive list of the enzymes and substrates useful in this invention.

Color producing moieties which are used to derivatize the above-mentioned substrates include: α and β naphthyl, α and β alkoxylnaphthyl (e.g. α and β 4-methoxy naphthyl), α and β 6-bromonaphthyl, o-nitrophenol, p-nitrophenol, 5-bromo-4-chloro-3-indolyl, bromothymolphthalein, phenolphthalein, 4-methylumbelliferyl, fluorescein, and the like.

**TABLE 2**

| Enzyme | Substrate |
|---|---|
| arylhydrolase | substituted hydrocarbons |
| arylsulfatase | sulfates |
| esterase | saturated and/or unsaturated, branched and/or straight-chain carboxylic acids of varying C numbers, and their salts (e.g. butyrate, acetate, caproate, caprylate, myristate, laureate, palmitate, oleate, valerate, etc.), phorbol esters |
| kinase | adenosine, adenosine triphosphate, cyclic adenosine monophosphate, guanosine diphosphate, cyclic guanosine monophosphate, phosphoserine, phosphotyrosine tetrazolium blue |
| lipase | glycerol fatty acid conjugate, phorbol esters |
| nuclease | thymidine-3-phosphate, polyinosinic-polycytidilic acid, polyadenylic-polyuridilic acid |
| oxidase | N,N-dimethyl-p-phenylene diamine HCl |
| peptidase | amino acid oligomers such as di- or tripeptides, amino acid naphthylamides |

Some of the enzyme substrate systems (e.g. aminopeptidases) require diazo coupling compounds for converting the reaction product to a chromophore. Such suitable diazo dyes include: 4-amino-2,5-dimethoxy-4'-nitroazobenzene diazonium salt; tetraazotized o-dianisidine; diazotized-4'-amino-2',5'-diethoxybenzanilide zinc chloride salt; diazotized product of 4 benzoylamino-2,5,-dimethoxyaniline zinc chloride; o-aminoazotoluene diazonium salt; anthraquinone-1-diazonium chloride; 5-nitro-2-amino-methoxybenzene diazotate; N',N'-diethyl-4-methoxymethanilamine diazonium salt; 2-amino-4-methoxybenzamide diazonium salt; diazo-2-amino-5-chloroanisole; diazo-5-chloro-o-anisidine; 5-chloro-2-toluidinediazonium chloride hemizinc chloride; 5-chloro-4-benzamido-2-methylbenzene diazonium chloride hemizinc chloride; 6-benzamido-5-methoxy-m-toluidine diazonium chloride, and other diazonium salts known in the art.

Alternate embodiments of this invention are directed to different ways of measuring the occurrence of the enzymatic reaction, which serves as an index of cell activation. In a preferred embodiment, a chromogenic substrate is acted upon by the enzyme of interest to yield a colored substrate, which can be measured as a fall in the absorbance at the appropriate wave-length of the plasma or medium in which the reaction takes place. Typical changes in 35 µl of cell rich plasma containing 30,000 ± 5000 blood cells is a decrease in absorbance of 0.100 ± 0.005 units in the presence of an activating agent compared to a background decrease of 0.001 ± 0.0005 units (based on a starting absorbance of 0.150 units). This measurement is preferably performed using a standard 96-well plate reader.

Together with the fall in plasma or medium absorbance is an increase in intracellular absorbance due to generation of the reaction product. Measurement of the intracellular color change can be performed together with the above measurement of the reaction fluid, or can be done as an alternative embodiment. The intracellular absorbance is preferably measured by scanning about 1000 cells in a well with a Hitachi Videomicroprobe (Inoue, S., Videomicroscopy, Plenum Press, N.Y., 1987) which emits a laser beam the transmission of which is blocked by the presence of colored reaction product within a cell. Thus, in this embodiment, "positive" cells can be enumerated and comparisons made for different cell types, different activating agents (e.g. antigens or allergens), etc.

The support matrix to which the cell-activating substance is bound can be the material of which the reaction vessel is constructed. That is, the reaction vessel itself, for example a modified 48 or 96 well microtitration plate, serves as the support matrix, with the activating substance bound to the bottom (and sidewalls) of the well. Materials of which the support matrix can be comprised include, but are not limited to, virgin optical styrene, substituted polystyrenes. acrylonitriles such as substituted acrylonitrile (SAN), polycarbonate, polypentene, or silicone oxide. In another embodiment, the support matrix made can be added separately to the reaction vessel, and comprises materials including, but not limited to, nitrocellulose, cellulose, polystyrene, styrene, SAN, polycarbonate, polypentene, divinyl benzene, or silicone oxide. The silicone oxide material useful for this invention is triple-siliconized glass or quartz. A preferred support matrix is virgin optical polystyrene which serves as the reaction vessel.

The microtiter plate in which the assay is conducted is important to the success of the assay method. The microtiter plate is designed to optimize physiological control of the reaction. It must allow adequate exchange of gases such as oxygen and CO₂, sufficiently rapid pH equilibration, adequate diffusion of molecules, ample dissipation of heat from exogenous sources or from endogenous reactions. Standard plastic 96-well microtitration plates, well known in the art, were found to be inadequate for the practice of this invention, largely due to the height of the sidewalls.

Thus, as previously mentioned, this invention is therefore also directed to a microtiter plate which has the following features:
(1) The ratio of the internal diameter of the wells (in mm) to reaction volume (in µl) must be greater than or equal to 0.1:1. Thus, for example, in a 6mm diameter microplate well, a 30µl sample volume is preferred.
(2) The side walls of the well should be as low as possible. For example a ratio of well height to reaction mixture height of 1.1:1 to 5:1 is acceptable with a ratio of about 2 being preferred.

The preferred well diameter is about 6 mm and the sidewall height is from 1-2 mm.

In one embodiment, the microtiter plate has the layout of a standard 96 well microtitration plate. In a preferred embodiment the microtiter plate has 48 wells (configured 8 by 6), equivalent in layout to half of a standard 96 well microtitration plate.

These microtiter plate layouts are particularly useful for measurement of chromogenic reactions, since one or two of the plates of this invention can be placed on top of standard optical quality 96 well plate so that the color reaction can be read in a typical spectrophotometric (or colorimetric) plate reader designed to accommodate standard 96 well microtitration plates.

The provision of cells for use in this invention requires that preparation and handling be minimal. This is important to prevent activation of various enzymes through stresses imposed on the cell in the preparation phase. For example, coagulation of blood, through the activation of serum kinases, complement components, Hagemann factors, etc., may lead to release of factors that may activate enzymes and raise background levels such that further activation in the assay does not result in a measurable increase in enzyme activity. Therefore it is important to avoid coagulation of blood. Excessive cell-cell contact such as caused by centrifugation, heat, or incubation leading to oxidative stress will also interfere in the successful operation of the assay. Therefore, one step preparation of cell-rich plasma, as described by Jaffe, R. et al. (J. Clin. Invest. 53:874-883 (1974)) is preferred.

Having now generally described the invention, the same will be more readily understood through reference to the following example which is provided by way of illustration, and is not intended to be limiting of the present invention, unless specified.

### EXAMPLE 1

Samples of blood were treated with 1/10 volume of 3.8% trisodium citrate, or with K,Mg,Na citrate, which contained an ene-diol compound, ascorbate, to maintain cellular oxidation-reduction potential, in order to prevent coagulation. The anticoagulated whole blood was centrifuged for a total of 980 gmin⁻¹. The cell-rich plasma (CRP) was aspirated using a plastic transfer pipet. The enzyme substrate, tetrazolium blue in a concentration range of between 100-10000 pmoles, in cell poor plasma, was added in 0.1 to 100 µl. (10 µl was found to be optimal.) Aliquots of 35 µl of CRP were transferred to virgin optical styrene 48 well microtiter plates as described above, to which antigens had been previously attached.

The cells were incubated for a range of times, between 2 and 24 hours at 35°C. (3 hours was found to be an optimal reaction time for most assays.) Cell activation was assessed as enzyme activity measured as a fall in color of the plasma. Absorbance was read at 340 nm or 340/380 nm on a BioRad Model 1500 plate reader.

The results following a 3 hour incubation at 35 ± 1° C were as follows:
A. The average intracellular absorbance (A₃₄₀) of lymphocytes (which included NK cells, CD4-positive T cells, null cells, and other lymphocytes) increased from a background of 0.204 ± .008 to 1.954 ± .051 units.
B. Absorbance of the reactant medium decreased by about 0.115 units (from 1.500 ± .001 to 1.385 ± .002 units) during this incubation.
C. Apparent cell volume for CD4-positive T lymphocytes increased from 6.4 ± 0.2 µ to 7.9 ± 0.22 µ (N=400, p < 0.001)

### EXAMPLE 2

To determine that the cells in a cell-rich plasma sample were directly responding to the activating agent, and to delineate which cells contributed, to the response, human lymphocytes were isolated from whole blood using isoosmotic isopycnic centrifugation on stractan^{tm} (arabinosyl-galactose) using standard gradient sedimentation techniques.

Cells were centrifuged at 15,000 gmin at 4°C in an IEC fixed angle rotor centrifuge. The recovered cells were >99% pure lymphocytes (with an occasional monocyte). Lymphocytes were resuspended in 5 ml Eagle's medium and subjected to a second stractan^{tm} gradient centrifugation.

The cells were incubated under conditions described in Example 1:
(1) 35 µl of cell suspension containing 30,000 ± 5000 cells, in Eagle's medium supplemented with either 10% fetal calf serum or human albumin.
(2) 180 min. incubation at 35°C, in a virgin optical styrene 48 well microtiter plate
(3) 10 µl of 0.1M tetrazolium blue was added as substrate.

Enzyme activation was observed both as an increase in apparent cell volume and an fall in absorbance of the incubation medium. These results indicate that the lymphocytes in the blood, and not serum components, or other cell types, were responsible for the enzymatic reaction.

### EXAMPLE 3

Antibodies specific for various lymphocyte classes were incorporated into the assay, as described above, to allow determination of the reacting cell types. This approach was based on the observation that antibodies specific to a particular cell type could selectively inhibit the activation of that cell, while permitting activation of other cells present.

Standard commercial monoclonal antibodies to the lymphocyte markers CD2, CD3, CD4, CD8, CD11, and CD12 were used. By adding the antibody to the reaction of cells, activating agent and substrate, it was determined that enzyme activation responses could be evoked in helper cells (CD4+), natural killer cells (CD12+) and "null" T cells (CD3+, CD4-, CD8-, CD11-, CD12-) These results indicate that certain T cell classes and natural killer cells were largely responsible for the response of the total lymphoid cell population to stimulation with antigens as shown in Table 1, to mitogenic lectins such as PHA and PWM, and to phorbol ester. Plasma cells, erythrocytes, and platelets did not contribute to the observed responses.

### EXAMPLE 4

To determine the predictive sensitivity and specificity of the assay using food and chemical allergens (as listed in Table 1) in the detection of late-phase or delayed hypersensitivity, results were classified according to the subjects' self-report of symptom frequency using a standard symptom questionnaire (the Cornell Medical Index / CMI^{R} / 1947, 1962, 1977). As shown below, subjects with few symptoms demonstrated few reactions. With progressively more symptoms, increasing numbers and intensity of reactions were noted in the assay.

| No. of Symptoms (by CMI) | Positive Reactions (out of 187 agents) |
|---|---|
| 0 | 0.6 ± 0.5 |
| 1 - 10 | 3.7 ± 0.9 |
| 11 - 20 | 5.8 ± 1.1 |
| 21 - 30 | 9.2 ± 1.2 |
| > 31 | 18.4 ± 8.4 |

To determine the effectiveness of using this assay under clinical conditions, a research follow-up study was conducted on a group of 94 subjects wherein assays were repeated at 6 month intervals. On repeat assay, a decrease in the number of substances to which the individual reacted was correlated with improvement as evidenced by reduction in symptom intensity reported by the subject. These results indicate a strong correlation between responsiveness to food or chemical antigenic stimuli in the assay of this invention and a clinically useful parameter.

### EXAMPLE 5

### A. Procedures

This analysis includes data from a total of 41 patients, undergoing 102 individual tests with retests spread out over a period of 7 to 32 months. Patients abstained from contact with the food and chemical agents to which they tested positively in the initial assay. The following data was obtained for each patient: time of test, gender, age, number of strong reactions, number of intermediate reactions, total number of reactions, and the test results (strong, medium, no reaction) for each of 180 foods and chemicals. Information on improvement during the months of observation consisted of the difference in the numbers of strong, intermediate, and total reactions for each individual between the first and last time the tests were taken. Also examined was the frequency of appearance of specific test agents, in order to identify and rank order the items with the greatest number of reactions.

### B. Results

The average overall reduction in positive reactions between the first and last tests show that the number of strong reactions were reduced by an average of 62%. Initially the average number of strong reactions was 29 (out of 180). On retest the average number of strong reactions dropped to 11. The average number of intermediate reactions increased from 11.3 to 18.2, which is attributable to the progression of strong reactions becoming less intense intermediate reactions before disappearing. In some cases new reactions appeared as a patient changed his or her diet. This accounts for a small portion of the increased number of intermediate reactions.

In sum, there was a reduction of almost two-thirds in the total number of strong reactions (p < 0.005). These results provide evidence that a significant reduction in the number of strong reactions can be expected by patients who remove from their diet or their environment those substances to which they initially reacted positively. The total number of reactions was also reduced. Symptomatic, clinical improvement occurred often before the detectable change in the activation response of the immune system.

For an examination of changes over time, patients who modified their diet/environment were grouped into three categories: those retested between 7 and 12 months, between 13 and 18 months, and after 19 months. Tests repeated within 6 months showed essentially the same reactions: a modest (3%) decrease in the total number of positive reactions observed on retest. This is consistent with the high precision and reproducibility of the assay procedure of this invention.

The shift from a state of hypersensitivity, wherein, cells of the immune system responded positively in the assay to specific antigens, to a state of non-reactivity to those antigens, appeared to require several months. Patients in this study typically had 3 to 20⁺ years of impaired function (i.e. hypersensitivity) and multiple, marginally or unsuccessful responses to treatment. More detailed, controlled studies are required before meaningful statements can be made about the total disappearance of reactions over time. Preliminary data indicate that the majority of reactions can be completely eliminated with careful modification of the patients' diet/environment.

Finally, reactions to specific foods and chemicals were classified as to frequency of reactions in this patient sample. The foods which are most common in our diet and most highly processed are the ones to which people most often become sensitized. Such items as corn products, chocolate/cocoa, nightshades, sugar, coffee, and yeast yield positive reactions about 70% of the time. Cow dairy products, including pasteurized cows' milk, casein, cheese, and butter gave reactions nearly 90% of the time. Clarified butter, which gave only a single positive reaction, lacks the immunologic reactants of its bovine source. It appears that the reactive protein and not the fat in dairy is responsible for the hypersensitivity. Also noteworthy was the 60% frequency of positive reactions to the fungus, Candida albicans, which is known to be clinically immunodepressing and has recently been implicated in a number of chronic illnesses.

### C. Conclusions

The use of the assay of this invention permits consistent quantitative assessment of foods and chemicals that cause immunological hypersensitivity reactions in sensitive individuals. The assay results can be translated into a clinically successful reversal of these reactions by appropriate modification of diet/environment. The results suggest that such reactions appear to be acquired and related to incomplete digestion and increased intestinal wall permeability associated with repeated consumption and are not, for the most part, genetic. These results therefore provide hope for those whose health has been adversely affected by hypersensitivity reactions linked to chronic illnesses and impaired immune defenses.

While this invention has been described in connection with specific embodiments thereof, it will be understood that it is capable of further modifications.

## Claims

1. An in vitro method of detecting the response of intact animal cells to an extra-cellular, exogenous cell activator substance, being a substance which, when in contact with the intact cells, activates an intracellular enzyme, which method comprises contacting the cells in vitro in the presence of a substrate for the activated enzyme with the said activator substance immobilised onto a solid support and detecting the consequent activity of the activated enzyme in the presence of the enzyme substrate, such contact taking place in one or more wells of a microtiter plate, characterised in that the well or wells of the microtiter plate, in which the contact takes place between the intact cells, the immobilised activator and the enzyme substrate has or have (each of them) a vertical side wall height of from 1 to 2 mm and the method is carried out such that a) the ratio of the internal diameter of the well (in mm.) to the volume of the sample (in µl.) is at least 0.1:1, and b) the ratio of the vertical height of the well to the vertical height (depth) of the sample in the well is in the range 1.1:1 to 5:1.

2. A method according to claim 1, characterised in that the ratio *of* the vertical height of the well to the vertical height (depth) of the sample in the well is about 2:1.

3. A method according to claim 1 or 2, characterised in that the internal diameter of the or each well is about 6mm.

4. A method according to any one of the preceding claims, wherein the activation of said enzyme by the exogenous cell activator substance is detected colorimetrically.

5. A method according to any one of the preceding claims wherein the said intracellular enzyme is an arylhydrolase, esterase, kinase, lipase, phosphatase, hexosaminidase, peptidase, or nuclease.

6. A method according to any one of the preceding claims, wherein the exogenous cell activator substance is immobilised onto the bottom and side walls of the well or wells of the microtiter plate.

7. A method according to any one of the preceding claims, wherein the intact cells are lymphocyte cells.

8. A method according to any one of the preceding claims, wherein the exogenous cell activator substance is an antigen, allergen or hapten.

9. A method according to claim 8, wherein the exogenous cell activator substance is phorbol ester, phytohemagglutinin, gluten or a sulfite.

10. A method according to any one of the preceding claims, which comprises simultaneously detecting the response of the intact cells to several different exogenous cell activator substances, wherein there is used a microtiter plate having the characteristics defined in any one of the preceding claims and containing two or more cell activator substances immobilised onto the bottom and sidewalls of the well or wells of the microtiter plate, each activator substance in a different well.

11. A method according to claim 1 wherein the intact animal cells are in the form of cell-rich plasma.

12. A microtiter plate for use in a method as claimed in any one of claims 1 to 11 wherein the well or wells of the microtiter plate contain therein intact animal cells and an immobilised exogenous cell activator substance which, when in contact with the intact cells, activates an intracellular enzyme characterised in that the well or wells of the microtiter plate in which the contact takes place between the intact cells, the immobilised activator and the enzyme substrate has or have (each of them) a vertical side wall height of from 1 to 2 mm and the method is carried out such that a) the ratio of the internal diameter of the well (in mm.) to the volume of the sample (in µl.) is at least 0.1:1, and b) the ratio of the vertical height of the well to the vertical height (depth) of the sample in the well is in the range 1.1:1 to 5:1.

13. A microtiter plate according to claim 12 wherein the intact animal cells, are in the form of cell rich plasma.

14. A microtiter plate according to claim 12 or claim 13 wherein the or each well has an internal diameter of about 6 mm.

15. A microtiter plate according to claim 14, having a cell activator substance immobilised onto the bottom and sidewalls of the wells thereof.

16. A microtiter plate according to claim 15, wherein the immobilised activator substance is an antigen, allergen or hapten.

17. A microtiter plate according to claim 16, wherein the immobilised activator substance is a substance as specified in claim 9.

18. A microtiter plate according to any one of claims 15 to 17 for use in a method according to claim 10, the said microtiter plate having two or more cell activator substances immobilised in the well or wells of the microtiter plate, each activator substance being in a different well.

19. A microtiter plate for use in a method as claimed in any one of claims 1 to 11, comprising a plurality of wells each having an internal diameter of about 6 mm and a side wall height in the range 1-2 mm and the method is carried out such that the well or wells of the microtiter plate in which the contact takes place between the intact cells, the immobilised activator and the enzyme substrate has or have (each of them) size dimensions in which a) the ratio of the internal diameter of the well (in mm.) to the volume of the sample (in µl.) is at least 0.1:1, and b) the ratio of the vertical height of the well to the vertical height (depth) of the sample in the well is in the range 1.1:1 to 5:1.

20. A microtiter plate according to claim 19, having a cell activator substance immobilised onto the bottom and sidewalls of the wells thereof.

21. A microtiter plate according to claim 20, wherein the immobilised activator substance is an antigen, allergen or hapten.

22. A microtiter plate according to claim 21, wherein the immobilised activator substance is a substance as specified in claim 9.

23. A microtiter plate according to any one of claims 20 to 22 for use in a method according to claim 10, the said microtiter plate having two or more cell activator substances immobilised onto the bottom and sidewalls of the well or wells of the microtiter plate, each activator substance being in a different well.

24. An assay kit for use in a method as claimed in any one of claims 1 to 11 comprising a microtiter plate as claimed in any one of claims 12 to 23 wherein the well or wells of the microtiter plate in which the contact takes place between the intact cells, the immobilised activator and the enzyme substrate has or have (each of them) a vertical side wall height of from 1 to 2 mm and the method is carried out such that a) the ratio of the internal diameter of the well (in mm.) to the volume of the sample (in µl.) is at least 0. 1:1, and b) the ratio of the vertical height of the well to the vertical height (depth) of the sample in the well is in the range 1.1:1 to 5:1.

25. An assay kit according to claim 24, which additionally includes a quantity of enzyme substrate appropriate to the enzyme activated by the said cell activator substance.

26. An assay kit according to claim 25, which additionally includes a quantity of a chromophoric reagent responsive to the activity of the enzyme when in the presence of the enzyme substrate and the immobilised cell activator substance.

## Patentansprüche

1. *In vitro*-Verfahren zur Feststellung der Reaktion intakter tierischer Zellen auf eine extrazelluläre exogene Zellaktivatorsubstanz, die eine Substanz ist, welche in Berührung mit den intakten Zellen ein intrazelluläres Enzym aktiviert, wobei man die Zellen *in vitro* in Gegenwart eines Substrates für das aktivierte Enzym mit der auf einem festen Träger mobilisierten Aktivatorsubstanz in Berührung bringt und die konsequente Aktivität des aktivierten Enzyms in Gegenwart des Enzymsubstrates feststellt, wobei diese Behandlung in einer oder mehreren Vertiefungen einer Miktrotiterplatte stattfindet, **dadurch gekennzeichnet**, daß die Vertiefung oder Vertiefungen der Mikrotiterplatte, in welchen die Berührung zwischen den intakten Zellen, dem immobilisierten Aktivator und dem Enzymsubstrat stattfindet, eine vertikale Seitenwandhöhe von 1 bis 2 mm hat bzw. haben (jede von ihnen) und daß das Verfahren so durchgeführt wird, daß
a) das Verhältnis des Innendurchmessers der Vertiefung (in mm) zu dem Volumen der Probe (in µl) wenigstens 0,1 : 1 ist und
b) das Verhältnis der vertikalen Höhe der Vertiefung zu der vertikalen Höhe (Tiefe) der Probe in der Vertiefung im Bereich von 1,1 : 1 bis 5 : 1 liegt.

2. Verfahren nach Anspruch 1, **dadurch gekennzeichnet**, daß das Verhältnis der vertikalen Höhe der Vertiefung zu der vertikalen Höhe (Tiefe) der Probe in der Vertiefung etwa 2 : 1 ist.

3. Verfahren nach Anspruch 1 oder 2, **dadurch gekennzeichnet**, daß der Innendurchmesser der oder jeder Vertiefung etwa 6 mm beträgt.

4. Verfahren nach einem der vorausgehenden Ansprüche, bei dem die Aktivierung des Enzyms durch die exogene Zellaktivatorsubstanz kolorimetrisch bestimmt wird.

5. Verfahren nach einem der vorausgehenden Ansprüche, bei dem das intrazelluläre Enzym eine Arylhydrolase, Esterase, Kinase, Lipase, Phosphatase, Hexosaminidase, Peptidase oder Nuclease ist.

6. Verfahren nach einem der vorausgehenden Ansprüche, bei dem die exogene Zellaktivatorsubstanz auf dem Boden und den Seitenwänden der Vertiefung oder Vertiefungen der Miktrotiterplatte immobilisiert ist.

7. Verfahren nach einem der vorausgehenden Ansprüche, bei dem die intakten Zellen Lymphozytzellen sind.

8. Verfahren nach einem der vorausgehenden Ansprüche, bei dem die exogene Zellaktivatorsubstanz ein Antigen, Allergen oder Hapten ist.

9. Verfahren nach Anspruch 8, bei dem die exogene Zellaktivatorsubstanz Phorbolester, Phytohämagglutinin, Gluten oder ein Sulfit ist.

10. Verfahren nach einem der vorausgehenden Ansprüche, bei dem man gleichzeitig die Reaktion der intakten Zellen auf mehrere verschiedene exogene Zellaktivatorsubstanzen bestimmt, wobei man eine Mikrotiterplatte mit den in einem der vorausgehenden Ansprüche gekennzeichneten Merkmalen verwendet, die zwei oder mehr auf dem Boden und den Seitenwänden der Vertiefung oder Vertiefungen der Miktrotiterplatte immobilisierte Zellaktivatorsubstanzen enthält, wobei jede Aktivatorsubstanz in einer anderen Vertiefung ist.

11. Verfahren nach Anspruch 1, bei dem die intakten tierischen Zellen in der Form von zellreichem Plasma vorliegen.

12. Mikrotiterplatte für die Verwendung bei einem Verfahren nach einem der Ansprüche 1 bis 11, bei der die Vertiefung oder Vertiefungen der Mikrotiterplatte darin intakte tierische Zellen und eine immobilisierte exogene Zellaktivatorsubstanz enthält bzw. enthalten, welche in Berührung mit den intakten Zellen ein intrazelluläres Enzym aktiviert, **dadurch gekennzeichnet,** daß die Vertiefung oder Vertiefungen der Mikrotiterplatte, in welcher bzw. welchen die Berührung zwischen den intakten Zellen, dem immobilisierten Aktivator und dem Enzymsubstrat stattfindet, eine vertikale Seitenwandhöhe von 1 bis 2 mm hat bzw. haben (jede von ihnen) und daß das Verfahren derart durchgeführt wird, daß
a) das Verhältnis des Innendurchmessers der Vertiefung (in mm) zu dem Volumen der Probe (in µl) wenigstens 0,1 : 1 ist und
b) das Verhältnis der vertikalen Höhe der Vertiefung zu der vertikalen Höhe (Tiefe) der Probe in der Vertiefung im Bereich von 1,1 : 1 bis 5 : 1 liegt.

13. Mikrotiterplatte nach Anspruch 12, bei der die intakten tierischen Zellen in der Form von zellreichem Plasma vorliegen.

14. Mikrotiterplatte nach Anspruch 12 oder 13, bei der die oder jede Vertiefung einen Innendurchmesser von etwa 6 mm hat.

15. Mikrotiterplatte nach Anspruch 14 mit einer auf dem Boden und den Seitenwänden ihrer Vertiefungen immobilisierten Zellaktivatorsubstanz.

16. Mikrotiterplatte nach Anspruch 15, bei der die immobilisierte Aktivatorsubstanz ein Antigen, Allergen oder Hapten ist.

17. Mikrotiterplatte nach Anspruch 16, bei der die immobilisierte Aktivatorsubstanz eine in Anspruch 9 angegebene Substanz ist.

18. Mikrotiterplatte nach einem der Anspruch 15 bis 17 für die Verwendung in einem Verfahren nach Anspruch 10, wobei die Mikrotiterplatte zwei oder mehr in der Vertiefung oder den Vertiefungen der Mikrotiterplatte immobilisierte Zellaktivatorsubstanzen hat, wobei sich jede Aktivatorsubstanz in einer anderen Vertiefung befindet.

19. Mikrotiterplatte für die Verwendung in einem Verfahren nach einem der Ansprüche 1 bis 11 mit mehreren Vertiefungen, von denen jede einen Innendurchmesser von etwa 6 mm und eine Seitenwandhöhe im Bereich von 1 bis 2 mm hat und das Verfahren derart durchgeführt wird, daß die Vertiefung oder Vertiefungen der Mikrotiterplatte, in welcher bzw. welchen die Berührung zwischen den intakten Zellen, dem immobilisierten Aktivator und dem Enzymsubstrat stattfindet (jede von ihnen), Größenabmessungen hat bzw. haben, bei denen
a) das Verhältnis des Innendurchmessers der Vertiefung (in mm) zu dem Volumen der Proben (in µl) wenigstens 0,1 : 1 beträgt und
b) das Verhältnis der vertikalen Höhe der Vertiefung zu der vertikalen Höhe (Tiefe) der Probe in der Vertiefung im Bereich von 1,1 : 1 bis 5 : 1 liegt.

20. Mikrotiterplatte nach Anspruch 19 mit einer auf dem Boden und den Seitenwänden ihrer Vertiefungen immobilisierten Zellaktivatorsubstanz.

21. Mikrotiterplatte nach Anspruch 20, bei der die immobilisierte Aktivatorsubstanz ein Antigen, Allergen oder Hapten ist.

22. Mikrotiterplatte nach Anspruch 21, bei der die immobilisierte Aktivatorsubstanz eine in Anspruch 9 angegebene Substanz ist.

23. Mikrotiterplatte nach einem der Ansprüche 20 bis 22 für die Verwendung in einem Verfahren nach Anspruch 10, wobei diese Mikrotiterplatte zwei oder mehr auf dem Boden und den Seitenwänden der Vertiefung oder Vertiefungen der Mikrotiterplatte immobilisierte Zellaktivatorsubstanzen aufweist, wobei sich jede Aktivatorsubstanz in einer anderen Vertiefung befindet.

24. Testsatz für die Verwendung in einem Verfahren nach einem der Ansprüche 1 bis 11 mit einer Mikrotiterplatte nach einem der Ansprüche 12 bis 23, bei der die Vertiefung oder Vertiefungen der Mikrotiterplatte, in welcher bzw. welchen die Berührung zwischen den intakten Zellen, dem immobilisierten Aktivator und dem Enzymsubstrat stattfindet (jede von ihnen) eine vertikale Seitenwandhöhe von 1 bis 2 mm hat und das Verfahren derart durchgeführt wird, daß
a) das Verhältnis des Innendurchmessers der Vertiefung (in mm) zu dem Volumen der Probe (in µl) wenigstens 0,1 : 1 ist und
b) das Verhältnis der vertikalen Höhe der Vertiefung zu der vertikalen Höhe (Tiefe) der Probe in der Vertiefung im Bereich von 1,1 : 1 bis 5 : 1 liegt.

25. Testsatz nach Anspruch 24, welcher zusätzlich eine für das durch die Zellaktivatorsubstanz aktivierte Enzym passende Enzymsubstratmenge enthält.

26. Testsatz nach Anspruch 25, welcher zusätzlich eine Menge eines chromophoren Reagenz enthält, das auf die Aktivität des Enzyms in Gegenwart des Enzymsubstrates und der immobilisierten Zellaktivatorsubstanz anspricht.

## Revendications

1. Procédé de détection in vitro de la réponse de cellules animales intactes à une substance exogène extracellulaire activant les cellules, substance qui, lorsqu'elle est au contact des cellules intactes, active une enzyme intracellulaire, ledit procédé comprenant les étapes consistant à amener les cellules in vitro, en présence d'un substrat pour l'enzyme activée, au contact de ladite substance activante immobilisée sur un support solide et à détecter l'activité conséquente de l'enzyme activée en présence du substrat enzymatique, un tel contact s'effectuant dans un ou plusieurs puits d'une plaque de microtitrage, caractérisé en ce que le puits ou les puits de la plaque de microtitrage dans lesquels s'effectue le contact entre les cellules intactes, l'activateur immobilisé et le substrat enzymatique a ou ont (chacun) une paroi latérale de 1 à 2 mm de hauteur, et on met le procédé en oeuvre de telle façon que a) le rapport du diamètre interne du puits (en mm) au volume de l'échantillon (en µl) soit d'au moins 0,1:1, et b) le rapport de la hauteur du puits à la hauteur (épaisseur) de l'échantillon dans le puits soit compris entre 1,1:1 et 5:1.

2. Procédé selon la revendication 1, caractérisé en ce que le rapport de la hauteur du puits à la hauteur (épaisseur) de l'échantillon dans le puits est d'environ 2:1.

3. Procédé selon la revendication 1 ou 2, caractérisé en ce que le diamètre interne du puits ou de chaque puits est d'environ 6 mm.

4. Procédé selon l'une quelconque des revendications précédentes, dans lequel l'activation de ladite enzyme par la substance exogène activant les cellules est détectée par colorimétrie.

5. Procédé selon l'une quelconque des revendications précédentes, dans lequel ladite enzyme intracellulaire est une arylhydrolase, une estérase, une kinase, une lipase, une phosphatase, une hexosaminidase, une peptidase ou une nucléase.

6. Procédé selon l'une quelconque des revendications précédentes, dans lequel la substance exogène activant les cellules est immobilisée au fond et sur les parois latérales du puits ou des puits de la plaque de microtitrage.

7. Procédé selon l'une quelconque des revendications précédentes, dans lequel les cellules intactes sont des cellules lymphocytes.

8. Procédé selon l'une quelconque des revendications précédentes, dans lequel la substance exogène activant les cellules est un antigène, un allergène ou un haptène.

9. Procédé selon la revendication 8, dans lequel la substance exogène activant les cellules est un ester de phorbol, la phytohémagglutinine, du gluten ou un sulfite.

10. Procédé selon l'une quelconque des revendications précédentes, comprenant la détection simultanée de la réponse des cellules intactes à plusieurs substances exogènes différentes activant les cellules, dans lequel on utilise une plaque de microtitrage ayant les caractéristiques définies dans l'une quelconque des revendications précédentes et contenant deux ou plus de deux substances d'activation cellulaire immobilisées au fond et sur les parois latérales du puits ou des puits de la plaque de microtitrage, chaque substance activante étant dans un puits différent.

11. Procédé selon la revendication 1, dans lequel les cellules animales intactes sont sous la forme d'un plasma riche en cellules.

12. Plaque de microtitrage destinée à une utilisation dans le procédé selon l'une quelconque des revendications 1 à 11, dans laquelle le puits ou les puits de la plaque de microtitrage contiennent des cellules animales intactes et une substance exogène immobilisée activant les cellules, qui, lorsqu'elle est au contact des cellules intactes, active une enzyme intracellulaire, caractérisée en ce que le puits ou les puits de la plaque de microtitrage dans lesquels s'effectue le contact entre les cellules intactes, l'activateur immobilisé et le substrat enzymatique a ou ont (chacun) des parois latérales de 1 à 2 mm de hauteur, et on met le procédé en oeuvre de telle façon que a) le rapport du diamètre interne du puits (en mm) au volume de l'échantillon (en µl) soit d'au moins 0,1:1, et b) le rapport de la hauteur du puits à la hauteur (épaisseur) de l'échantillon dans le puits soit compris entre 1,1:1 et 5:1.

13. Plaque de microtitrage selon la revendication 12, dans laquelle les cellules animales intactes sont sous la forme d'un plasma riche en cellules.

14. Plaque de microtitrage selon la revendication 12 ou la revendication 13, dans laquelle le puits ou chaque puits a un diamètre interne d'environ 6 mm.

15. Plaque de microtitrage selon la revendication 14, ayant une substance d'activation cellulaire immobilisée au fond et sur les parois latérales des puits de celle-ci.

16. Plaque de microtitrage selon la revendication 15, dans laquelle la substance activante immobilisée est un antigène, un allergène ou un haptène.

17. Plaque de microtitrage selon la revendication 16, dans laquelle la substance activante immobilisée est une substance telle que spécifiée dans la revendication 9.

18. Plaque de microtitrage selon l'une quelconque des revendications 15 à 17 pour une utilisation dans un procédé selon la revendication 10, ladite plaque de microtitrage ayant deux ou plus de deux substances d'activation cellulaire qui sont immobilisées dans le puits ou dans les puits de la plaque de microtitrage, chaque substance activante étant dans un puits différent.

19. Plaque de microtitrage pour une utilisation dans un procédé selon l'une quelconque des revendications 1 à 11, comprenant une pluralité de puits ayant chacun un diamètre interne d'environ 6 mm et des parois latérales d'une hauteur comprise entre 1 et 2 mm, et on met le procédé en oeuvre de telle façon que le puits ou les puits de la plaque de microtitrage dans lesquels s'effectue le contact entre les cellules intactes, l'activateur immobilisé et le substrat enzymatique ait ou aient (chacun) des dimensions telles que a) le rapport du diamètre interne du puits (en mm) au volume de l'échantillon (en µl) soit d'au moins 0,1:1, et b) le rapport de la hauteur du puits à la hauteur (épaisseur) de l'échantillon dans le puits soit compris entre 1,1:1 et 5:1.

20. Plaque de microtitrage selon la revendication 19, ayant une substance d'activation cellulaire immobilisée au fond et sur les parois latérales des puits de celle-ci.

21. Plaque de microtitrage selon la revendication 20, dans laquelle la substance activante immobilisée est un antigène, un allergène ou un haptène.

22. Plaque de microtitrage selon la revendication 21, dans laquelle la substance activante immobilisée est une substance telle que spécifiée dans la revendication 9.

23. Plaque de microtitrage selon l'une quelconque des revendications 20 à 22 pour une utilisation dans un procédé selon la revendication 10, ladite plaque de microtitrage ayant deux ou plus de deux substances d'activation cellulaire immobilisées au fond et sur les parois latérales du puits ou des puits de la plaque de microtitrage, chaque substance activante étant dans un puits différent.

24. Coffret d'analyse pour une utilisation dans un procédé selon l'une quelconque des revendications 1 à 11, comprenant une plaque de microtitrage selon l'une quelconque des revendications 12 à 23, dans lequel le puits ou les puits de la plaque de microtitrage dans lequel/lesquels s'effectue le contact entre les cellules intactes, l'activateur immobilisé et le substrat enzymatique a ou ont (chacun) des parois latérales de 1 à 2 mm de hauteur, et on met le procédé en oeuvre de telle façon que a) le rapport du diamètre interne du puits (en mm) au volume de l'échantillon (en µl) soit d'au moins 0,1:1, et b) le rapport de la hauteur du puits à la hauteur (épaisseur) de l'échantillon dans le puits soit compris entre 1,1:1 et 5:1.

25. Coffret d'analyse selon la revendication 24, comprenant, en outre, une quantité de substrat enzymatique appropriée à l'enzyme activée par ladite substance activant les cellules.

26. Coffret d'analyse selon la revendication 25, comprenant, en outre, une quantité d'agent chromophore sensible à l'activité de l'enzyme quand il est en présence du substrat enzymatique et de la substance immobilisée activant les cellules.
